# EUROPEAN PATENT SPECIFICATION

(11) **EP 1 817 073 B1**
(45) Date of publication and mention of the grant of the patent: **17.01.2018**
(21) Application number: 05824009.4
(22) Date of filing: 14.11.2005
(51) Int. Cl.: A61M 29/00, A61B 18/14

(54) **IMPROVED ENERGY DELIVERY DEVICES**
VERBESSERTE VORRICHTUNGEN
DISPOSITIFS AMELIORES D'APPORT D'ENERGIE

(30) Priority: 12.11.2004 US 627662 P; 21.10.2005 US 256295
(43) Date of publication of application: 15.08.2007
(73) Proprietor: Boston Scientific Scimed, Inc., Maple Grove, MN 55311 (US)
(72) Inventor: DALBEC, Tim, R., Saratoga, CA 95070 (US); KAPLAN, Gary, S., Mountain View, California 94040 (US); DANEK, Christopher, J., San Carlos, CA 94070 (US); WIZEMAN, William, J., Mountain View, CA 94041 (US)
(74) Representative: Vossius & Partner Patentanwälte Rechtsanwälte mbB
(86) International application number: PCT/US2005/041244
(87) International publication number: WO 2006/053309

(56) References cited:
- US-A- 6 014 579
- US-A- 6 119 030
- US-A1- 2003 233 099
- US-B1- 6 411 852

## Description

### BACKGROUND OF THE INVENTION

Asthma is a disease in which (i) bronchoconstriction, (ii) excessive mucus production, and (iii) inflammation and swelling of airways occur, causing widespread but variable airflow obstruction thereby making it difficult for the asthma sufferer to breathe. Asthma is a chronic disorder, primarily characterized by persistent airway inflammation. However, asthma is further characterized by acute episodes of additional airway narrowing via contraction of hyper-responsive airway smooth muscle.

Asthma is managed pharmacologically by: (1) long term control through use of anti-inflammatories and long-acting bronchodilators and (2) short term management of acute exacerbations through use of short-acting bronchodilators. Both of these approaches require repeated and regular use of the prescribed drugs. High doses of corticosteroid anti-inflammatory drugs can have serious side effects that require careful management. In addition, some patients are resistant to steroid treatment. The difficulty involved in patient compliance with pharmacologic management and the difficulty of avoiding stimulus that triggers asthma are common barriers to successful asthma management.

Current management techniques are neither completely successful nor free from side effects. Presently, a new treatment for asthma is showing promise. This treatment comprises the application of energy to the airway smooth muscle tissue. Additional information about this treatment may be found in commonly assigned patents and applications in U.S. patent nos. 6,411,852, 6,634,363 and U.S. published application nos. US-2005-0010270-A1 and US-2002-0091379-A1.

The application of energy to airway smooth muscle tissue, when performed via insertion of a treatment device into the bronchial passageways, requires navigation through tortuous anatomy as well as the ability to treat a variety of sizes of bronchial passageways. As discussed in the above referenced patents and applications, use of an RF energy delivery device is one means of treating smooth muscle tissue within the bronchial passageways.

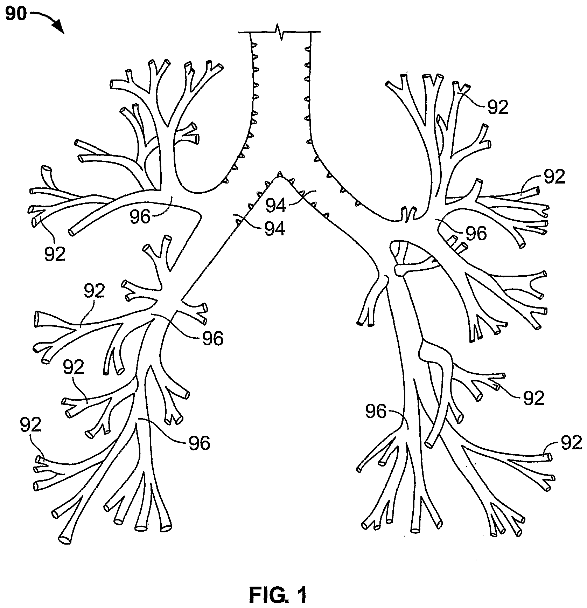

Fig. 1A illustrates a bronchial tree 90. As noted herein, devices treating areas of the lungs must have a construction that enables navigation through the tortuous passages. As shown, the various bronchioles 92 decrease in size and have many branches as they extend into the right and left bronchi 94. Accordingly, an efficient treatment requires devices that are able to treat airways of varying sizes as well as function properly when repeatedly deployed after navigating through the tortuous anatomy.

Tortuous anatomy also poses challenges when the treatment device requires mechanical actuation of the treatment portion (e.g., expansion of a treatment element at a remote site). In particular, attempting to actuate a member may be difficult in view of the fact that the force applied at the operator's hand-piece must translate to the distal end of the device. The strain on the operator is further intensified given that the operator must actuate the distal end of the device many times to treat various portions of the anatomy. When a typical device is contorted after being advanced to a remote site in the lungs, the resistance within the device may be amplified given that internal components are forced together.

It is also noted that the friction of polymers is different from that of metals. Most polymers are viscoelastic and deform to a greater degree under load than metals. Accordingly, when energy or force is applied to move two polymers against each other, a significant part of friction between the polymers is the energy loss through inelastic hysteresis. In addition, adhesion between polymers also plays a significant part in the friction between such polymers.

In addition to basic considerations of navigation and site access, there exists the matter of device orientation and tissue contact at the treatment site. Many treatment devices make contact or are placed in close proximity to the target tissue. Yet, variances in the construction of the treatment device may hinder proper alignment or orientation of the device. For example, in the case of a device having a basket-type energy transfer element that is deployed intralumenally, the treatment may benefit from uniform contact of basket elements around the perimeter of the lumen. However, in this case, design or manufacturing variances may tend to produce a device where the angle between basket elements is not uniform. This problem tends to be exacerbated after repeated actuation of the device and/or navigating the device through tortuous anatomy when the imperfections of the device become worsened through plastic deformation of the individual components. Experience demonstrates that once a member becomes predisposed to splaying (i.e., not maintaining the desired angular separation from an adjacent element), or inverting (i.e., buckling inward instead of deploying outward), the problem is unlikely to resolve itself without requiring attention by the operator. As a result, the operator is forced to remove the device from the patient, make adjustments, and then restart treatment. This interruption tends to increase the time of the treatment session.

As one example, commonly assigned U.S. Patent Number 6,411,852 describes a treatment for asthma using devices having flexible electrode members that can be expanded to better fill a space (e.g., the lumen of an airway.) However, the tortuous nature of the airways was found to cause significant bending and/or flexure of the distal end of the device. As a result, the spacing of electrode members tended not to be even. In some extreme cases, electrode elements could tend to invert, where instead of expanding an electrode leg would invert behind an opposing leg.

For many treatment devices, the distortion of the energy transfer elements might cause variability in the treatment effect. For example, many RF devices heat tissue based on the tissue's resistive properties. Increasing or decreasing the surface contact between the electrode and tissue often increases or decreases the amount of current flowing through the tissue at the point of contact. This directly affects the extent to which the tissue is heated. Similar concerns may also arise with resistive heating elements, devices used to cool the airway wall by removing heat, or any energy transfer device. In any number of cases, variability of the energy transfer/tissue interface causes variability in treatment results. The consequential risks range from an ineffective treatment to the possibility of patient injury.

Furthermore, most medical practitioners recognize the importance of establishing acceptable contact between the transfer element and tissue. Therefore, distortion of the transfer element or elements increases the procedure time when the practitioner spends an inordinate amount of time adjusting a device to compensate for or avoid such distortion. Such action becomes increasingly problematic in those cases where proper patient management limits the time available for the procedure.

For example, if a patient requires an increasing amount of medication (e.g., sedatives or anesthesia) to remain under continued control for performance of the procedure, then a medical practitioner may limit the procedure time rather than risk overmedicating the patient. As a result, rather than treating the patient continuously to complete the procedure, the practitioner may plan to break the procedure in two or more sessions. Subsequently, increasing the number of sessions poses additional consequences on the part of the patient in cost, the residual effects of any medication, adverse effects of the non-therapeutic portion of the procedure, etc.

In addition to the above, because the procedure is generally performed under direct visualization via a scope-type device, it may be desirable for a medical practitioner to directly observe the treatment areas so that the next adjacent area of tissue may be treated while minimizing overlap between treatment areas. Alternatively, or in combination, the medical practitioner may advance a device out of the bronchoscope into distal airways where visualization is difficult because the scope's light source is insufficient or blocked. Accordingly, there remains a need to provide a device that supplements the illumination provided by the scope, or illuminates the airway with a light of a particular wavelength that allows the practitioner to better observe the treatment area.

In view of the above, the present methods and devices described herein provide an improved means for treating tortuous anatomy such as the bronchial passages. It is noted that the improvements of the present device may be beneficial for use in other parts of the anatomy as well as the lungs. Document US-A-2003/233099 discloses the most relevant prior art.

### SUMMARY OF THE INVENTION

The present invention includes devices configured to treat the airways or other anatomical structures, and may be especially useful in tortuous anatomy. The devices described herein are configured to treat with uniform or predictable contact (or near contact) between an active element and tissue. Typically, the invention allows this result with little or no effort by a physician. Accordingly, aspects of the invention offer increased effectiveness and efficiency in carrying out a medical procedure. The increases in effectiveness and efficiency may be especially apparent in using devices having relatively longer active end members.

In view of the above, a variation of the disclosure includes a catheter for use with a power supply, the catheter comprising a flexible elongate shaft coupled to at least one energy transfer element that is adapted to apply energy to the body lumen. The shaft will have a flexibility to accommodate navigation through tortuous anatomy. The energy transfer elements are described below and include basket type design, or other expandable designs that permit reduction in size or profile to aid in advancing the device to a particular treatment site and then may be expanded to properly treat the target site. The basket type designs may be combined with expandable balloon or other similar structures.

Variations of the device can include an elongate sheath having a near end, a far end adapted for insertion into the body, and having a flexibility to accommodate navigation through tortuous anatomy, the sheath having a passageway extending therethrough, the passageway having a lubricious layer extending from at least a portion of the near end to the far end of the sheath. Where the shaft is slidably located within the passageway of the sheath.

Variations of devices described herein can include a connector for coupling the energy transfer element to the power supply. The connector may be any type of connector commonly used in such applications. Furthermore, the connector may include a cable that is hard-wired to the catheter and connects to a remote power supply. Alternatively, the connector may be an interface that connects to a cable from the power supply.

As noted below, variations of the device allow for reduce friction between the shaft and sheath to allow relatively low force advancement of a distal end of the shaft out of the far end of the sheath for advancement the energy transfer element.

Additional variations of the disclosure include devices allowing for repeatable deployment of the expandable energy transfer element while maintaining the orientation and/or profile of the components of the energy transfer element. One such example includes an energy transfer basket comprising a plurality of legs, each leg having a distal end and a proximal end, each leg having a flexure length that is less than a full length of the leg. The legs are coupled to near and far alignment components. The near alignment component includes a plurality of near seats extending along an axis of the alignment component. The near alignment component can be secured to the elongate shaft of the device. The far alignment component may have a plurality of far seats extending along an axis of the alignment component, where the plurality of near seats are in alignment with the plurality of far seats. In these variations of the device, each distal end of each leg is nested within a far seat of the far alignment component and each proximal end of each leg is nested within a near seat of the near alignment component such that an angle between adjacent legs is determined by an angle between adjacent near seats and the flexure length of each length is determined by the distance between near and far alignment components.

One or both of the components may include stops that control flexure length of each leg. Such a design increases the likelihood that the flexure of each leg is uniform.

An additional variation of the device includes a catheter for use in tortuous anatomy to deliver energy from a power supply to a body passageway. Such a catheter includes an expandable energy transfer element having a reduced profile for advancement and an expanded profile to contact a surface of the body passageway and an elongate shaft having a near end, a far end adapted for insertion into the body, the expandable energy transfer element coupled to the far end of the shaft, the shaft having a length sufficient to access remote areas in the anatomy. The design of this shaft includes a column strength sufficient to advance the expandable energy transfer element within the anatomy, and a flexibility that permits self-centering of the energy transfer element when expanded to contact the surface of the body passageway.

In a further variation of the disclosure, the device and/or system may include an illumination source and/or supply. The illumination source may be configured to provide a single or multiple wavelength of light depending upon the particular application. For example, the device may be configured to provide illumination that is visible light, or white light. The illumination can be a single visible color such as red, green, blue, yellow, or a combination. The illumination may be a non-visible wavelength that is made visible by some type of filter or other such means on the scope or viewing monitor for the scope.

When tissue, in particular airway wall tissue, is heated as a result of treatment, collagen fibers within the tissue loose their organization. As a result, the ability to polarize transmitted and reflected light is altered. In some cases, depending on temperature, the polarization axis changes. This is a so-called change in birefringence. In certain cases, tissue heated to a sufficiently high temperature may lose the ability to polarize light. Therefore, the illumination may be suited to view areas of heated collagen fibers so as to identify treated tissue (e.g., with the procedures described in the patents discussed above and 6,634,363, US publication 20020091379A1). Various wavelengths (including but not limited to wavelengths in the infrared, ultraviolet, as well as visible spectrum) of the illumination source and/or filters may be used so that the medical practitioner may identify the treated tissue.

In addition, certain wavelengths may afford separation from red and orange (e.g., 590 nm, 570 nm, 470 nm or yellow, green, and blue.) These colors may offer better distinction when used in airways.

### BRIEF DESCRIPTION OF THE DRAWINGS

Each of the following figures diagrammatically illustrates aspects of the invention. Variation of the invention from the aspects shown in the figures is contemplated.
Fig. 1 is an illustration of the airways within a human lung.
Fig. 2A is a schematic view of an exemplary system for delivering energy according to the present invention.
Fig. 2B is a side view of a device extending out of an endoscope/bronchoscope, where the device has an active distal end for treating tissue using energy delivery.
Figs. 3A-3G show various features of the device allowing for low force deployment of the energy element.
Figs. 4A-4C illustrate various alignment components of the device.
Figs. 4D-4E demonstrate the alignment components coupled to a leg of the device.
Figs. 4F-4H illustrate an additional variation of an alignment component.
Figs. 5A-5B is a variation of an energy transfer element according to the present device.
Figs. 5C-5D show variations in which the legs of the device are biased to expand outward.
Figs. 6A-6C show various basket configurations for the device.
Figs. 7A-7D illustrate various features of variations of legs for use with the present devices.
Figs. 8A-8D show various junctions for use with the present devices to improve alignment when the device is advanced through tortuous anatomy.
Figs. 9A-9J are addition variations of junctions.
Figs. 10A-10D shows additional variations of junctions for use in the present devices.
Figs. 11A-10C shows additional variations of systems and devices using illumination.

### DETAILED DESCRIPTION

It is understood that the examples below discuss uses in the airways of the lungs. However, unless specifically noted, the invention is not limited to use in the lung. Instead, the invention may have applicability in various parts of the body. Moreover, the invention may be used in various procedures where the benefits of the device are desired.

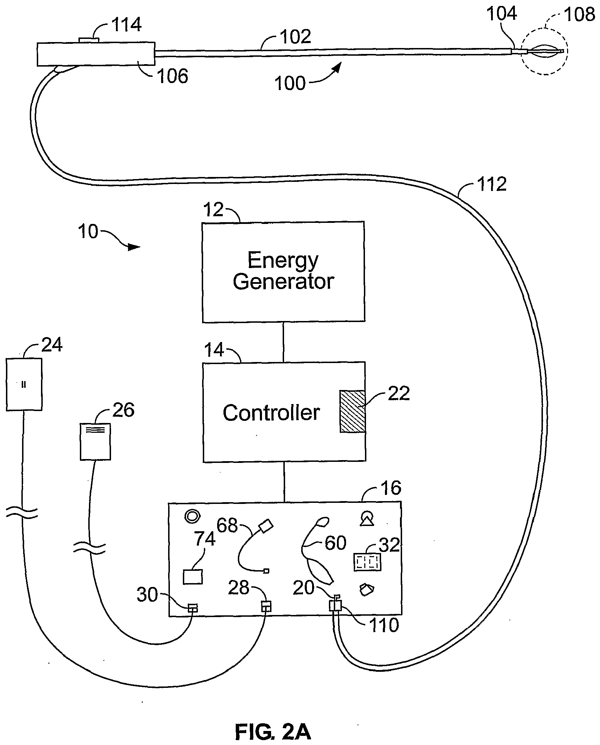

Fig. 2A shows a schematic diagram of one example of a system **10** for delivering therapeutic energy to tissue of a patient for use with the device described herein. The illustrated variation shows, the system **10** having a power supply (e.g., consisting of an energy generator **12,** a controller **14** coupled to the energy generator, a user interface surface **16** in communication with the controller **14).** It is noted that the device may be used with a variety of systems (having the same or different components). For example, although variations of the device shall be described as RF energy delivery devices, variations of the device may include resistive heating systems, infrared heating elements, microwave energy systems, focused ultrasound, cryo-ablation, or any other energy deliver system. It is noted that the devices described should have sufficient length to access the tissue targeted for treatment. For example, it is presently believed necessary to treat airways as small as 3 mm in diameter to treat enough airways for the patient to benefit from the described treatment (however, it is noted that the invention is not limited to any particular size of airways and airways smaller than 3 mm may be treated). Accordingly, devices for treating the lungs must be sufficiently long to reach deep enough into the lungs to treat these airways. Accordingly, the length of the sheath/shaft of the device that is designed for use in the lungs should preferably be between 1.5-3 ft long in order to reach the targeted airways.

The particular system **10** depicted in Fig. 2A is one having a user interface as well as safety algorithms that are useful for the asthma treatment discussed above. Addition information on such a system may be found in U.S. Provisional application No.: 60/674,106, filed April 21, 2005 entitled CONTROL METHODS AND DEVICES FOR ENERGY DELIVERY.

Referring again to Fig. 2A, a variation of a device **100** described herein includes a flexible sheath **102,** an elongate shaft **104** (in this example, the shaft extends out from the distal end of the sheath **102),** and a handle or other operator interface **106** (optional) secured to a proximal end of the sheath **102.** The distal portion of the device **100** includes an energy transfer element **108** (e.g., an electrode, a basket electrode, a resistive heating element, cyroprobe, etc.). Additionally, the device includes a connector **110** common to such energy delivery devices. The connector **110** may be integral to the end of a cable **112** as shown, or the connector **110** may be fitted to receive a separate cable **112.** In any case, the device is configured for attachment to the power supply via some type connector **110.** The elongate portions of the device **102** and **104** may also be configured and sized to permit passage through the working lumen of a commercially available bronchoscope or endoscope. As discussed herein, the device is often used within an endoscope, bronchoscope or similar device. However, the device may also be advanced into the body with or without a steerable catheter, in a minimally invasive procedure or in an open surgical procedure, and with or without the guidance of various vision or imaging systems.

Fig. 2A also illustrates additional components used in variations of the system. Although the depicted systems are shown as RF type energy delivery systems, it is noted that the invention is not limited as such. Other energy delivery configurations contemplated may include or not require some of the elements described below. The power supply (usually the user interface portion **16)** shall have connections **20, 28, 30** for the device **100,** return electrode **24** (if the system **10** employs a monopolor RF configuration), and actuation pedal(s) **26** (optional). The power supply and controller may also be configured to deliver RF energy to an energy transfer element configured for bipolar RF energy delivery. The user interface **16** may also include visual prompts **32, 60, 68, 74** for user feedback regarding setup or operation of the system. The user interface **16** may also employ graphical representations of components of the system, audio tone generators, as well as other features to assist the user with system use.

In many variations of the system, the controller **14** includes a processor **22** that is generally configured to accept information from the system and system components, and process the information according to various algorithms to produce control signals for controlling the energy generator **12.** The processor **22** may also accept information from the system **10** and system components, process the information according to various algorithms and produce information signals that may be directed to the visual indicators, digital display or audio tone generator of the user interface in order to inform the user of the system status, component status, procedure status or any other useful information that is being monitored by the system. The processor **22** of the controller **14** may be digital IC processor, analog processor or any other suitable logic or control system that carries out the control algorithms. U.S. Provisional application no. 60/674,106 filed April 21, 2005 entitled CONTROL METHODS AND DEVICES FOR ENERGY DELIVERY.

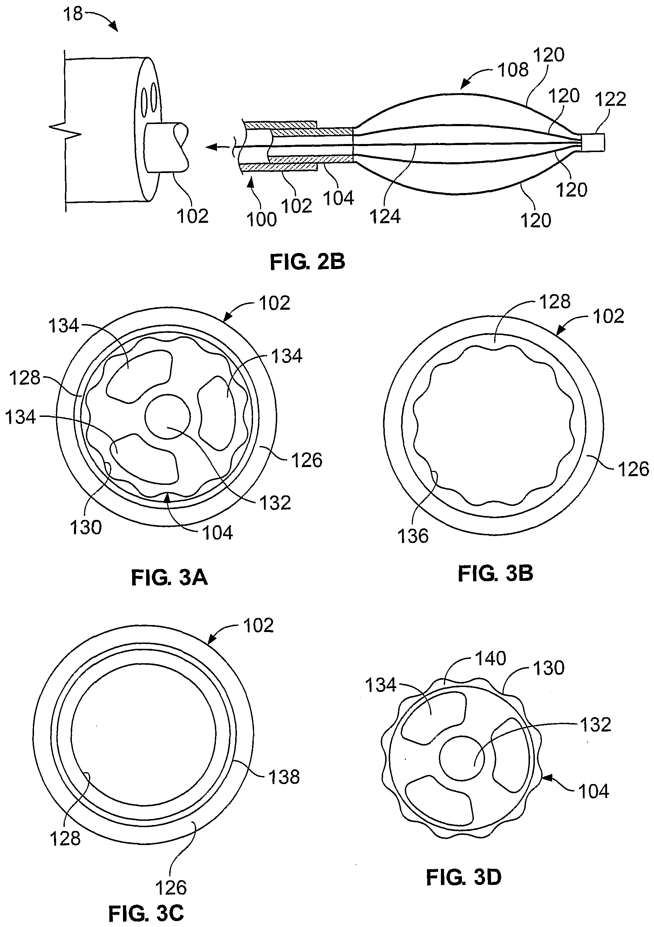

Fig. 2B illustrates one example of an energy transfer element **108.** In this example the energy transfer element **108** is a "basket-type" configuration that requires actuation for expansion of the basket in diameter. Such a feature is useful when the device is operated intralumenally or in anatomy such as the lungs due to the varying size of the bronchial passageways that may require treatment. As illustrated, the basket contains a number of arms **120** which carry electrodes (not shown). In this variation the arms **120** are attached to the elongated shaft **104** at a proximal end while the distal end of the arms **120** are affixed to a distal tip **122.** To actuate the basket **108** a wire or tether **124** is affixed to the distal tip **122** to enable compression of the arms **120** between the distal tip **122** and elongate sheath **104.**

Fig. 2B also illustrates the device **100** as being advanced through a working channel **32** of a bronchoscope **18.** While a bronchoscope **18** may assist in the procedure, the device **100** may be used through direct insertion or other insertion means as well.

As noted above, some variations of the devices described herein have sufficient lengths to reach remote parts of the body (e.g., bronchial passageways around 3mm in diameter). Figs. 3A-3G illustrate various configurations that reduce the force required to actuate the device's basket or other energy transfer element.

Fig. 3A illustrates a cross section taken from the sheath **102** and elongate shaft **104.** As shown, the sheath **102** includes an outer layer **126** and an inner lubricious layer **128.** The outer layer **126** may be any commonly known polymer such as Nylon, PTFE, etc. The lubricious layers **128** discussed herein may comprise a lubricious polymer (for example, HDPE, hydrogel, polytetrafluoroethylene). Typically, lubricious layer **128** will be selected for optimal pairing with the shaft **104.** One means to select a pairing of polymers is to maximize the difference in Gibbs surface energy between the two contact layers. Such polymers may also be chose to give the lubricious layer **128** a different modulus of elasticity than the outer layer **126.** For example, the modulus of the lubricious layer **128** may be higher or lower than that of the outer layer **126.**

Alternatively, or in combination, the lubricious layers may comprise a fluid or liquid (e.g., silicone, petroleum based oils, food based oils, saline, etc.) that is either coated or sprayed on the interface of the shaft **104** and sheath **102.** The coating may be applied at the time of manufacture or at time of use. Moreover, the lubricious layers **128** may even include polymers that are treated such that the surface properties of the polymer changes while the bulk properties of the polymer are unaffected (e.g., via a process of plasma surface modification on polymer, fluoropolymer, and other materials). Another feature of the treatment is to treat the surfaces of the devices with substances that provide antibacterial/antimicrobial properties.

In one variation of the invention, the shaft **104** and/or sheath **102** will be selected from a material to provide sufficient column strength to advance the expandable energy transfer element within the anatomy. Furthermore, the materials and or design of the shaft/sheath will permit a flexibility that allows the energy transfer element to essentially self-align or self-center when expanded to contact the surface of the body passageway. For example, when advanced through tortuous anatomy, the flexibility of this variation should be sufficient that when the energy transfer element expands, the shaft and/or sheath deforms to permit self-centering of the energy transfer element. It is noted that the other material selection and/or designs described herein shall aid in providing this feature of the invention.

Fig. 3A also depicts a variation of a shaft **104** for use in the present device. In this variation the shaft **104** includes a corrugated surface **130.** It is envisioned that the corrugated surface **130** may include ribbed, textured, scalloped, striated, ribbed, undercut, polygonal, or any similar geometry resulting in a reduced area of surface contact with any adjoining surface(s). The corrugated surface **130** may extend over a portion or the entire length of the shaft **104.** In addition, the shape of the corrugations may change at varying points along the shaft **104.**

The shaft **104** may also include one or more lumens **132, 134.** Typically, one lumen will suffice to provide power to the energy transfer elements (as discussed below). However, in the variation show, the shaft may also benefit from additional lumens (such as lumens **134)** to support additional features of the device (e.g., temperature sensing elements, other sensor elements such as pressure or fluid sensors, utilizing different lumens for different sensor leads, and fluid delivery or suctioning, etc.). In addition the lumens may be used to deliver fluids or suction fluid to assist in managing the moisture within the passageway. Such management may optimize the electrical coupling of the electrode to the tissue (by, for example, altering impedance). Since the device is suited for use in tortuous anatomy, a variation of the shaft **104** may have lumens **134** that are symmetrically formed about an axis of the shaft. As shown, the additional lumens **134** are symmetric about the shaft **104.** This construction provides the shaft **104** with a cross sectional symmetry that aid in preventing the shaft **104** from being predisposed to flex or bend in any one particular direction.

Fig. 3B illustrates another variation where the sheath **102** includes an outer layer **126** and a lubricious layer **128.** However, in this variation the lubricious layer **128** also includes a corrugated surface **136.** It is noted that any combination of the sheath **102** and shaft **104** may have a corrugated surface.

Fig. 3C illustrates yet another aspect of construction of a sheath **102** for use with the present device. In this variation, the sheath **102** includes a multi-layer construction having an outer layer **126,** one or more middle layers **138.** The middle layers **138** may be selected to have properties that transition between the outer layer properties and the lubricious layer properties, and improve the bonding between inner and outer layer. Alternatively, the middle layer **138** may be selected to aid in the column strength of the device. An example of the middle layer includes Plexar PX 306, 3060, and/or 3080.

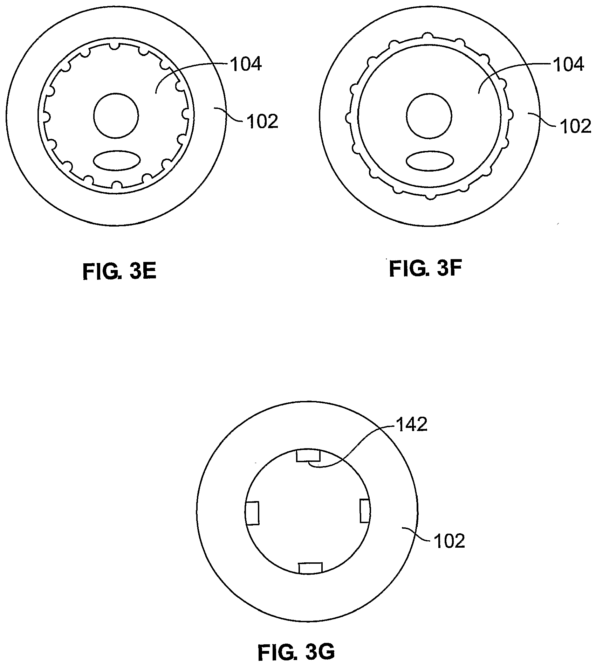

Fig. 3D depicts a variation of a shaft **104** for use with the devices described herein where the shaft outer surface comprises a lubricious layer **140.** As illustrated, the shaft outer surface may also optionally have a corrugated surface **130.** Figs. 3E-3G illustrate additional variations of corrugated surfaces. As shown in Fig. 3E and 3F, either or both the sheath **102** and the shaft **104** may have corrugated surfaces that are formed by interrupting the surface. Naturally, different shapes and configurations may be otherwise constructed. Fig. 3G illustrates a variation where the sheath **102** comprises protrusions or spacer **142** to separate the surfaces of the sheath/shaft.

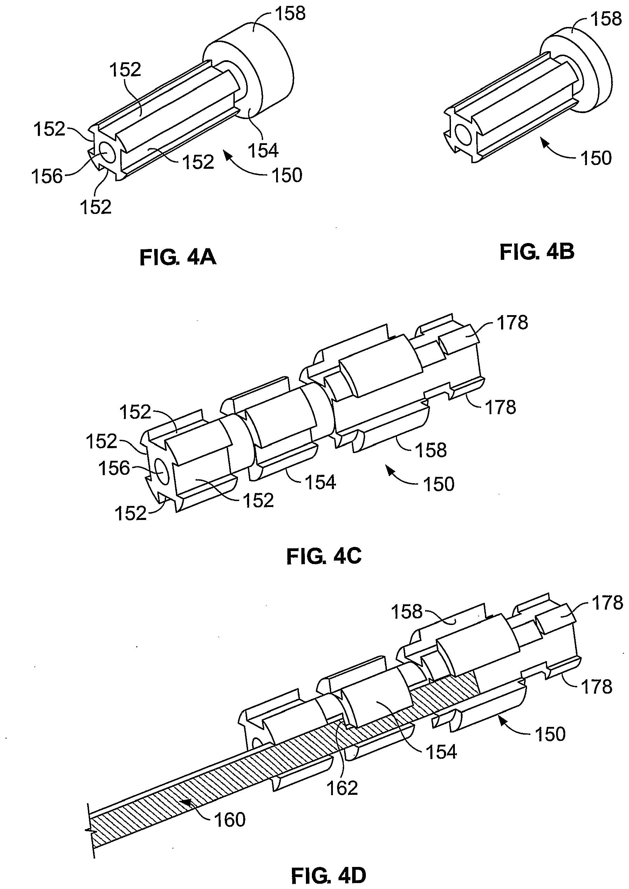

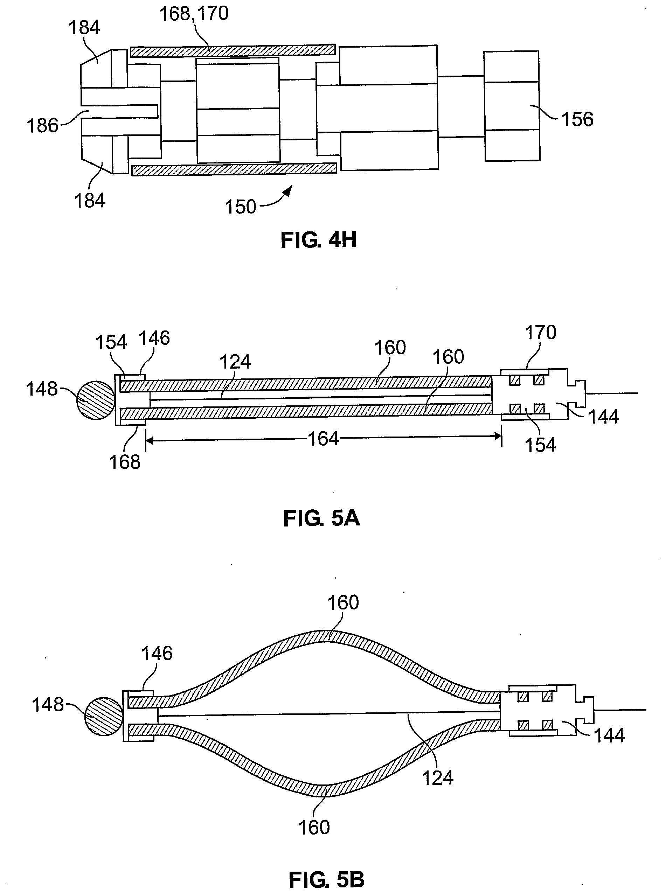

Figures 4A-4D illustrate yet another feature that may be incorporated with any of the subject devices. Figure 4A illustrates an example of an alignment component **150.** In this variation, the alignment component **150** includes a plurality of seats **152** that nest electrode arms (not shown). As discussed herein, the seats **152** allow for improved control of the angular spacing of the arms. Moreover, the seats **152** permits design of a device in which the flexure length of each of the arms of a basket type device is uniform (even if the tolerance of each arm varies). Though the alignment component **150** is shown as having four seats **152,** any number of seats may be employed.

The alignment component **150** also includes a stop **154.** The stop **154** acts as a reference guide for placement of the arms as discussed below. In this variation, the stop **154** is formed from a surface of an end portion **158.** This end portion **158** is typically used to secure the alignment component **150** to (or within) the sheath/shaft of the device. The alignment component **150** may optionally include a through hole or lumen **156.**

Fig. 4B illustrates another variation of an alignment component **152.** This variation is similar to the variation shown in Fig. 4A, with the difference being the length of the end portion **158.** The smaller end portion **158** may optionally be employed when the component **150** is used at the distal end of the device. In such a case, the component **158** may not be attached to the sheath or shaft. In addition, the end portion **158** may optionally be rounded, for example, to minimize tissue trauma that may be caused by the end of the device.

The alignment components **150** of the present invention may be fabricated from a variety of polymers (such as nylon or any other polymer commonly used in medical devices), either by machining, molding, or by cutting an extruded profile to length. One feature of this design is electrical isolation between the legs, which may also be obtained using a variation of the invention that employs a ceramic material for the alignment component. However, in one variation of the invention, an alignment component may be fabricated from a conductive material (e.g., stainless steel, polymer loaded with conductive material, or metallized ceramic) so that it provides electrical conductivity between adjacent electrode legs. In such a case, a power supply may be coupled to the alignment component, which then electrically couples all of the legs placed in contact with that component. The legs may be attached to the conductive alignment component with conductive adhesive, or by soldering or welding the legs to the alignment component. This does not preclude the legs and alignment component form being formed from one piece of metal.

Devices of the present invention may have one or more alignment components. Typically the alignment components are of the same size and/or the angular spacing of the seats is the same. However, variations may require alignment components of different sizes and/or different angular spacing. Another variation of the invention is to have the seats at an angle relative to the axis of the device, so as to form a helically shaped energy delivery element.

Fig. 4C illustrates another variation of an alignment component **150.** In this variation, the alignment component **150** includes four seats **152.** This variation includes an alignment stop **154** that protrudes from the surface of the component **150.** In addition, the end portion **158** of the alignment component **150** is also of a cross section that may improve strength of the connection between the component and the sheath/shaft. In this case, the end portion **158** allows for crimping of the sheath/shaft. Optionally as shown, radial protrusions **178** at the right of the end portion **158** may be included to allow heat bonding of the alignment component to the shaft. In this case, the shaft may be a polymer with a melting temperature lower than that of the alignment component. When constrained to be coaxial, heat, and if necessary axial pressure, may be applied to join the two components.

Fig. 4D illustrates the protrusion-type stop **154** that retains a notch **162** of the electrode leg **160.** This mode of securing the electrode leg **160** provides a "redundant-type" joint. In one example, the leg **160** is secured to the alignment component **150** using a sleeve (not shown) placed over both the leg **160** and alignment component **150** with or without the use of an adhesive within the sleeve. The notch **162** in the leg **160** is placed around the protrusion-type-stop **154.** As a result, the notch-stop interface prevents the leg from being pulled from the device and is especially useful to prevent the proximal or near ends of the legs from separating from the device. It is noted that this safety feature is especially important when considering that if the proximal/near ends of the legs separate and hook on the anatomical passage, it may be difficult or impossible to remove the device from the passage. Such a failure may require significant medical intervention.

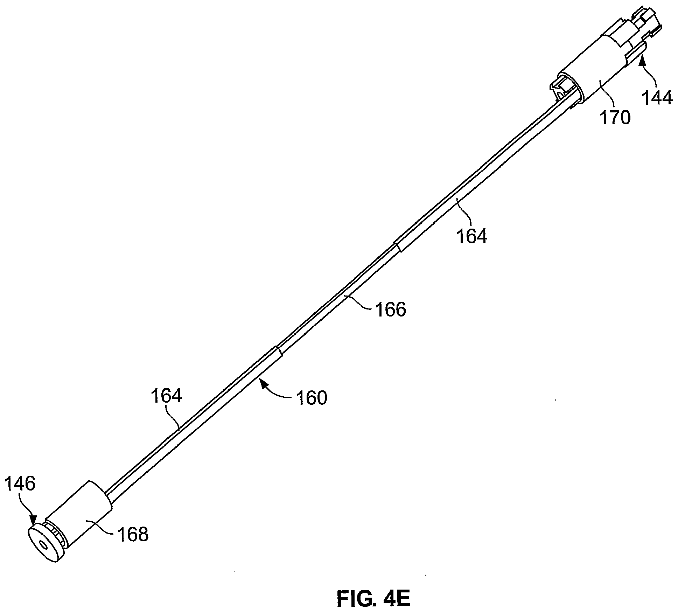

Fig. 4E illustrates one example of a leg **160** affixed to near/proximal and far/distal alignment components **144, 146.** As shown, the leg **160** may have an insulated portion **164** and an exposed portion **166** that form electrodes. The near and far ends of the leg **160** are secured to respective alignment components **144, 146.** In this example, sleeves **168** and **170** cover the leg and alignment component. As noted above, one or both of the alignment components may be electrically conductive to provide power to the electrodes. Furthermore, adhesive (e.g., cyanoacrylate, UV-cured acrylic, epoxy, or any such adhesive) may also be used to secure the leg to the components.

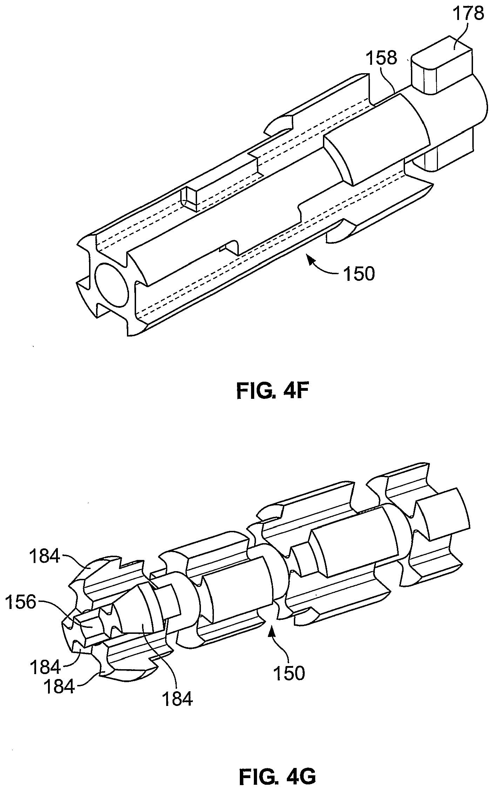

Additionally, the alignment components may be designed such that the sleeves may be press or snap fit onto the alignment components, eliminating the need for adhesively bonding the sleeves to the alignment components. Fig. 4F illustrates a perspective view of an end portion of an alignment component **150** having one or more slots **186** to create end portion segments **184.** The slots **186** permit deflection of the end portion segments **184** to allow sliding of a sleeve or hypotube (either a near or far sleeve **168** or **170)** over the end portion. Fig. 4G shows a cross sectional view of the component **150** of Fig. 4F. As shown, once advanced over the end portion segment **184,** the sleeve or hypotube becomes secured to the component **150.** To lock the sleeve in place, an insert or wire member **124** (not shown) is placed in the through hole or lumen **156.** The insert or wire member prevents inward deflection of the end portion segments **184** thereby ensuring that the sleeve or hypotube remains secured to the component **150.**

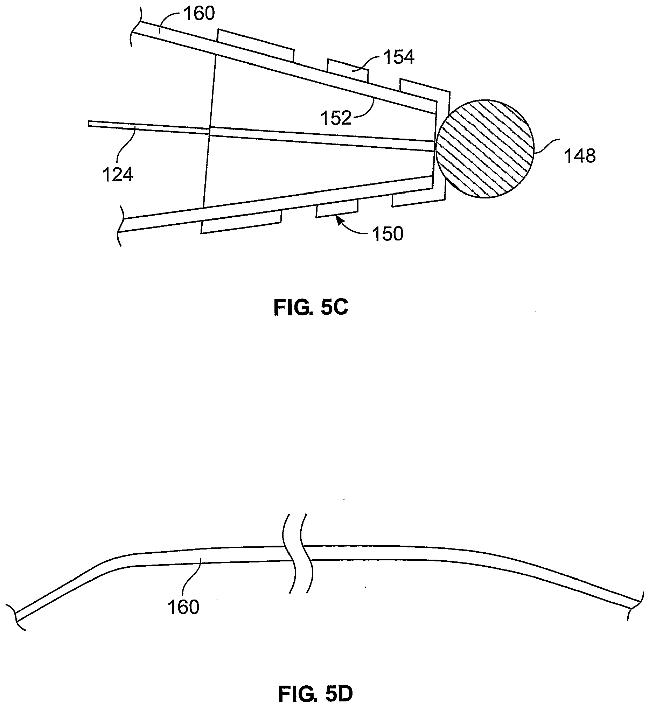

Fig. 5A shows a cross sectional view of two legs **160** attached to alignment components **144, 146.** The sheath and shaft have been omitted for clarity. The flexure length **164** of the leg **160** is defined as the length between the alignment components **144, 146** over which the leg may flex when the proximal and distal ends are moved closer to one another. As noted above, the alignment components permit the flexure length **164** of the legs **160** to be uniform even if the leg lengths vary. The flexure length **164** is essentially set by the longest leg, the shorter legs may float between the stops **154** of the alignment components **144, 146.** As an additional measure to prevent the legs **160** from inverting, the lengths of the sleeves **168** and **170** may be selected to be less than the length of the respective alignment components **144, 146** (as shown in the figure). The tendency of the leg to deflect outward can be improved by selecting the sleeve length as such. When the legs **160** expand they are supported by their respective seat on the interior side but unsupported on outer side. In yet another variation, the the seats can slant to predispose the arms to deflect in a desired direction. For example, as shown in Fig. 5C, the seats **152** can slant as shown to predispose the legs **160** to outward deflection. Such a construction can be accomplished by machining or by drafting a molded part in the direction of the catheter axis. As shown in Fig. 5D, the leg can have a slight bend or shape that predisposes the legs to bow outward.

Fig. 5B illustrates the variation of Fig. 5A in an expanded state. As shown, the device may have a wire **124** or other similar member that permits movement of the far alignment component **146** relative to the near alignment component **144.** As noted herein, the wire **124** may be electrically conductive to provide power to electrodes on the device. Fig. 5B also illustrates a ball tip **148** at the end of the device. The ball tip **148** may serve as a means to secure the wire **124** as well as providing an atraumatic tip for the device.

Variations of the wire **124** may include a braided or coiled wire. The wire may be polymer coated or otherwise treated to electrically insulate or increase lubricity for easier movement within the device.

To expand the energy transfer element **108,** the wire **124** may be affixed to a handle **106** and actuated with a slide mechanism **114** (as shown in Fig. 2A.) In an alternative design, the wire **124** may be affixed between the handle **106** and the distal end of the energy transfer element **108.** In such a case, the slide mechanism **114** may be affixed to the shaft **104.** Movement of the slide mechanism **114** causes expansion of the element **108** as the shaft causes movement of the proximal end of the energy transfer element (being fixed to the shaft) relative to the distal end of the energy transfer element (being fixed to the wire **124.** In an additional variation, movement of the slide **114** may have two outcomes: 1) advancing the energy transfer element out of the sheath; and 2) subsequently expanding the energy transfer element. Such constructions are disclosed in U.S. Patent Application No. 09/436,455 filed November 8, 1999.

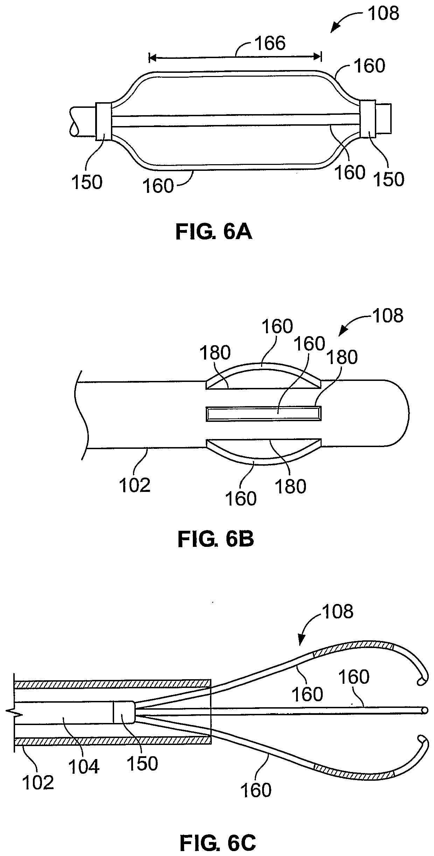

Fig. 6A illustrates a variation of an energy transfer element **108** in which the legs **160** have a pre-determined shape. This shape may be selected as required for the particular application. As shown, the predetermined shape provides a certain length of the electrode **166** that may be useful for treatment of a long section of tissue.

Fig. 6B illustrates another variation of the energy transfer element **108.** In this variation, the legs **160** extend out of openings **180** in the sheath **102** (in other variations, the legs may extend out of openings in the shaft). Accordingly, the alignment components and other parts of the device would be located within the sheath **102.**

Fig. 6C illustrates yet another variation of an energy transfer element **108.** In this variation, the basket is connected at a proximal end and opened at a distal end. The electrode legs **160** only have a single alignment component **150.** The conductive member (or wire) may be located within the shaft **104.** In this variation, advancement of the energy transfer element **108** out of the sheath **102** causes expansion of the element. The energy transfer elements may be predisposed or spring loaded to bow outward when advanced from the sheath.

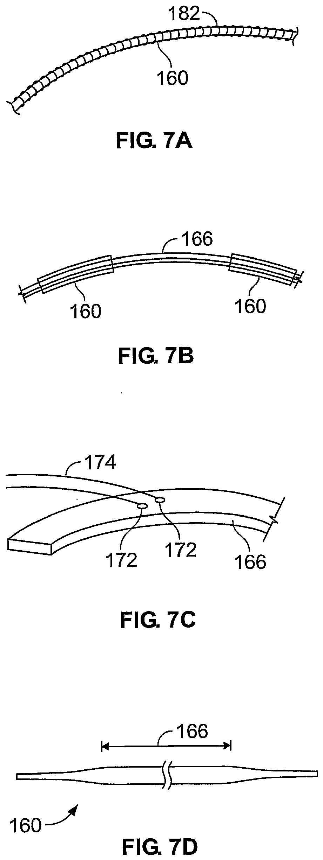

Fig. 7A illustrates an example of a leg **160** with an energy element **180** coiled around the leg **160.** In this example, the energy element **182** uses conductive heating and comprises a resistance heating element coiled around the leg **160.** Fig. 7B illustrates a variation of the invention having an RF electrode attached to the basket leg **160.** The RF electrode may be attached to the basket leg **160** via the use of a fastener. For example, the electrode may be attached via the use of a heat shrink fastener, (e.g., polymeric material such as PET or polyethylene tubing). Alternatively, as discussed above, the entire leg may be a conductive medium where a non-conductive coating insulates the majority of the leg leaving the electrode portion uninsulated. Further examples of energy transfer element configurations include paired bipolar electrodes, where the pairs are leg to leg or within each leg, and large matrices of paired electrodes affixed to a variety of expanding members (balloons, mechanisms, etc.)

Fig. 7C illustrates a variation of the invention having thermocouple leads **172** attached to an electrode **166** or leg of the device. The leads may be soldered, welded, or otherwise attached. This variation of the invention shows both leads **172** of the thermocouple **174** attached in electrical communication to a leg **160** at separate joints (or the leads may be separated but the solder on each connection actually flows together). In this case, the temperature sensor is at the surface of the leg. This variation provides a safety measure in case either joint becomes detached, the circuit will be open and the thermocouple **174** stops reading temperature. Such a condition may be monitored via the power supply and allow a safe shutdown of the system.

By spacing the leads of the thermocouple closely together to minimize temperature gradients in the energy transfer element between the thermocouple leads, thermoelectric voltage generated within the energy transfer element does not compromise the accuracy of the measurement. The leads may be spaced as close together as possible while still maintaining a gap so as to form an intrinsic junction with the energy transfer element. In another variation of the device, the thermocouple leads may be spaced anywhere along the tissue contacting region of the energy transfer element. Alternatively, or in combination, the leads may be spaced along the portion of an electrode that remains substantially straight. The intrinsic junction also provides a more accurate way of measuring surface temperature of the energy transfer element, as it minimizes the conduction error associated with an extrinsic junction adhered to the device.

The thermocouple leads may be attached to an interior of the leg or electrode. Such a configuration protects the thermocouple as the device expands against tissue and protects the tissue from potential trauma. The device may also include both of the thermocouple leads as having the same joint.

The devices of the present invention may use a variety of temperature sensing elements (a thermocouple being just one example, others include, infrared sensors, thermistors, resistance temperature detectors (RTDs), or any other component capable of detecting temperatures or changes in temperature). The temperature detecting elements may be placed on a single leg, on multiple legs or on all of the legs.

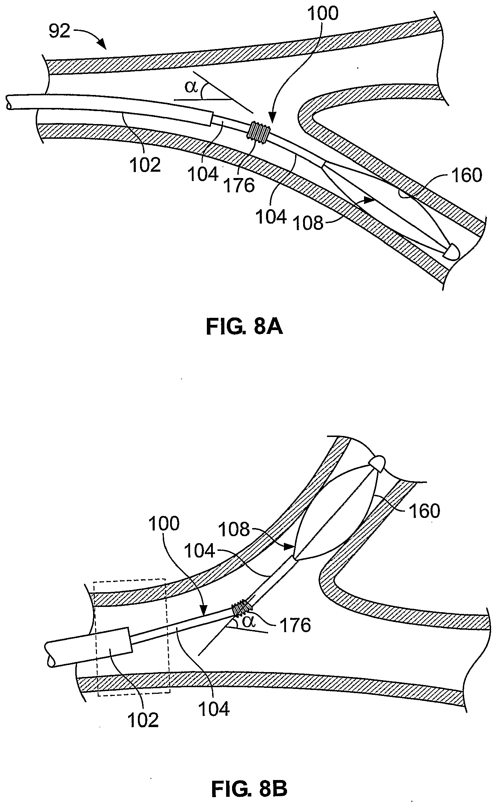

The present invention may also incorporate a junction that adjusts for misalignment between the branching airways or other body passages. This junction may be employed in addition to the other features described herein. Fig. 8A illustrates a device **100** having such a junction **176** allowing alignment of the device to closely match the alignment of the airway. It is noted that the present feature also benefits those cases in which the pathway and target site are offset as opposed to having an angular difference.

The junction **176** helps to eliminate the need for alignment of the axis of the active element **108** with the remainder of the device in order to provide substantially even tissue contact. The junction may be a joint, a flexure or equivalent means. A non-exhaustive listing of examples is provided below.

The legs **160** of the energy transfer element may have various shapes. For example, the shapes may be round, rounded or polygonal in cross section. Additionally, each leg may change cross section along its axis, providing for, for example, electrodes that are smaller or larger in cross section that the distal and proximal portions of each leg. This would provide a variety of energy delivery characteristics and bending profiles, allowing the design to be improved such that longer or wider electrode configurations can be employed. For example, as shown in Fig. 7D, if the cross-sectional thickness of the electrode portion **166** of the leg **160** is greater than the cross-sectional thickness of the distal and proximal portions of the leg, the leg would be predisposed to bow outward in the distal and proximal sections, while remaining flatter in the electrode area of the leg, potentially providing improved tissue contact.

As for the action the junction enables, it allows the distal end of the device to self-align with the cavity or passageway to be treated, irrespective of the alignment of the access passageway. Fig. 8A illustrates an example of where the access passageway and passageway to be treated are misaligned by an angle α. In the example shown in Fig. 8B, the misalignment angle α is greater than the angle illustrated in Fig. 8A. Yet, the energy transfer element **108** of the treatment device **100** remains substantially aligned with the target area.

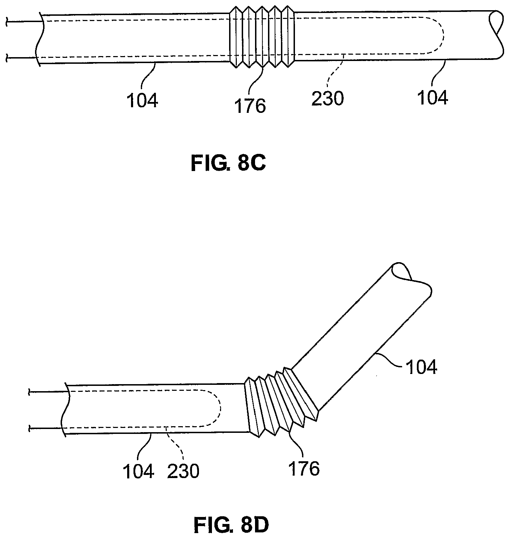

Figs. 8C and 8D illustrate an additional variation of the junction **176.** In this variation the junction **176** may be reinforced with a reinforcing member **230.** The reinforcing member may have some degree of flexibility to navigate the tortuous anatomy, but the flexibility will be less than the junction **176.** As shown in Fig. 8C, the reinforcing member **230** maintains the device shaft **104** in an aligned position, preferably for insertion, removal, and or navigation of the device. When desired, the reinforcing member **230** may be removed from the junction **176** as illustrated in fig. 8D. Accordingly, upon removal, the device is free to flex or orientate as desired. Furthermore, the reinforcing member may be reinserted within the junction **176** when repositioning or removing the device from the target site. In additional variations, it is contemplated that the reinforcing member may be placed external to the device/junction.

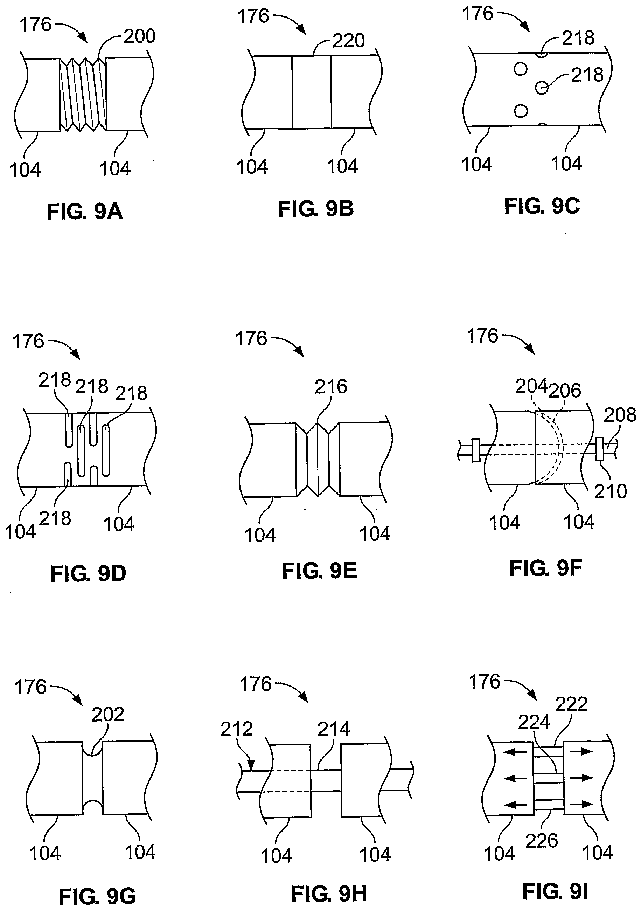

Figs. 9A-9I illustrate additional junctions for use in the devices described herein. As for these examples, Fig. 9A illustrates a junction **176** in the form of a plurality of turns or coils **200** of a spring. The coil offers a flexure with 3-dimensional freedom allowing realignment of the active end of the subject device in any direction. Of course, a simple hinge or universal joint may also be employed.

The length of the junction (whether a spring junction or some other structure) may vary. Its length may depend on the overall system diameter. It may also depend on the degree of compliance desired. For example, with a longer effective junction length (made by extending the coil with additional turns), the junction becomes less rigid or more "floppy".

In any case, it may be desired that the junction has substantially the same diameter of the device structure adjacent the junction. In this way, a more atraumatic system can be provided. In this respect, it may also be desired to encapsulate the junction with a sleeve or covering if they include open or openable structures. Junction **176** shown in Figs. 8A and 8B is illustrated as being covered. A covering can help avoid contaminating the joint with body fluid or debris which could compromise junction function.

Some of the junctions are inherently protected. Junction **176** shown in Fig. 9B comprises a polymer plug **220** or a section of polymer having a different flexibility or durometer than adjacent sections. When a separate piece of polymer is to be employed, it can be chemically, adhesively, or heat welded to adjacent structure; when the junction is formed integrally, this may be accomplished by selective vulcanizing, or reinforcement (even with a braid or by other means of forming a composite structure). Generally, it is noted that any connection of pieces or construction provided may be produced by methods known by those with skill in the art.

As for junction **176** shown in Fig. 9C, it is formed by removing sections of material from the body of the device. Openings **218** formed at the junction may be left empty, covered or filled with a more compliant material. Fig. 9D also shows a junction **176** in which openings are provided to provide increased flexibility. Here, openings **218** are offset from each other to form a sort of flexible universal joint. In either junction variation shown in Fig. 9C or 9D, the size, number shape, etc. of the opening may vary or be tuned as desired.

Fig. 9E shows a junction **176** in the form of a bellows comprising plurality of pleats **216.** Here too, the number of pleats, etc. may be varied to achieve desirable performance.

Junction **176** in Fig. 9F shows a true "joint" configuration. In this case, it is a universal joint provided by ball **204** and socket **206.** These elements may be held together by a tie wire **208,** possibly with caps **210.** Other configurations are possible as well.

Fig. 9G illustrates a junction **176** in the form of a reduced diameter section **202.** This variation offers greater flexibility by virtue of its decreased moment of inertia at the junction. While section **202** is integrally formed, the related junction **176** in Fig. 9H is formed from a hypotube or wire **212** having an exposed junction section **214** on the shaft **104.** Variations of the invention will permit a junction having a reduced bending moment of inertia section as compared to the remainder of the device and/or shaft of the device. Reducing the bending moment of inertia may be accomplished in any number of ways. For example, there could be an area of reduced diameter, a section of material having a lower modulus, a section having a different shape, a flexible joint structure, etc. It should be noted that there are many additional ways to reduce the bending moment that will be readily apparent to those skilled in the art viewing the invention disclosed herein.

Yet another junction example is provided in Fig. 9I. Here junction **176** comprises a plurality of wires **222, 224, 226.** In one variation, the wires simply offer increased flexibility of the junction. In another variation, the wires serve as an "active" or "dynamic" junction. The wires may be adjusted relative to one another to physically steer the distal end of the device. This junction may be manipulated manually with an appropriate user interface - especially one, like a joy-stick, that allows for full 3-dimensional or rotational freedom - or it may be controlled by automation using appropriate hardware and software controls. Of course, other "dynamic" junctions are possible as well.

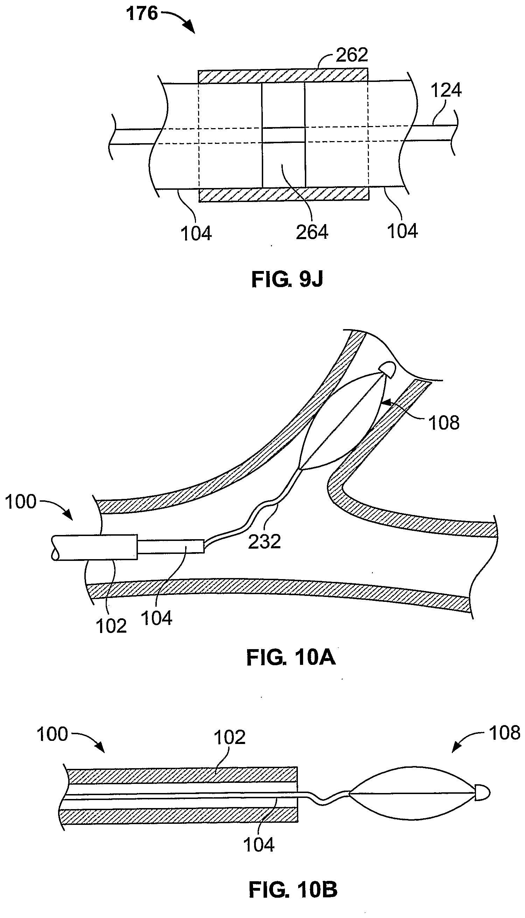

Fig. 9J shows another joint configuration **176** employing an external sleeve **262** between sections of the shaft **104.** A moveable wire **124** to actuate a distal basket or the like is also shown. The space between the wire and sleeve may be left open as shown, or filled in with a flexible polymer **264,** such as low durometer urethane, a visco-elastic material, etc. Though not necessary, providing an internal member may improve system pushability. The sleeve itself will typically be a polymeric sleeve. It may be heat-shrink material such as PET tubing; it may be integrally formed with either catheter body portion and press fit or slip fit and glued over other etc.

Another variation of the junctions includes junctions variations where the shaft **104** is "floppy" (i.e., without sufficient column strength for the device to be pushable for navigation). In Fig. 10A, a tether **232** connects energy transfer element **108** to the shaft **104** of the device **100.** The tether may simply comprise a flexible wire or cable, it may comprise a plurality of links, etc. The tether variation of the invention also accommodates relative motion between the device and the body (e.g., tidal motion of breathing, other muscle contractions, etc.) The tether permits the device to move relative to its intended treatment location unless the user desires and uses the tether or the sheath to pull the device back or drive it forward. The tether may have an alignment component (not illustrated) at the near end of the energy transfer element **108.**

To navigate such a device to a treatment site, the energy transfer element **108** and tether **232** may be next to or within the sheath **102.** In this manner, the column strength provided by the sheath allows for advancement of the active member within the subject anatomy.

The same action is required to navigate the device shown in Fig. 10B. What differs in this variation of the invention, however, is that the "tether" is actually a continuation of a highly flexible shaft **104.** In this case, the shaft **104** of the device is shown with a thicker or reinforced wall. In such a device, the shaft carries the compressive loads on the device back to its distal end.

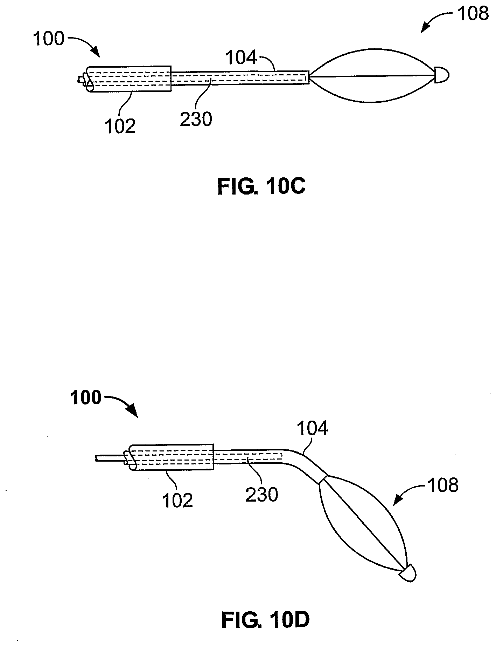

Like the device in Fig. 10B, the devices in Figs. 10C and 10D have highly flexible shafts **104.** However, instead of a stiffening external sheath, the device may employ a stiffening obturator **230** within a lumen of the shaft **104.** As shown in Fig. 10C, when the obturator **230** fills the lumen, the device is relatively straight or stiff. When the shaft is withdrawn as shown in Fig. 10D, the distal end of the device is "floppy" or easily conformable to the subject anatomy. With the shaft advanced substantially to the end of the device, it offers ease of navigation; when withdrawn, it offers a compliant section according to an aspect of the present invention.

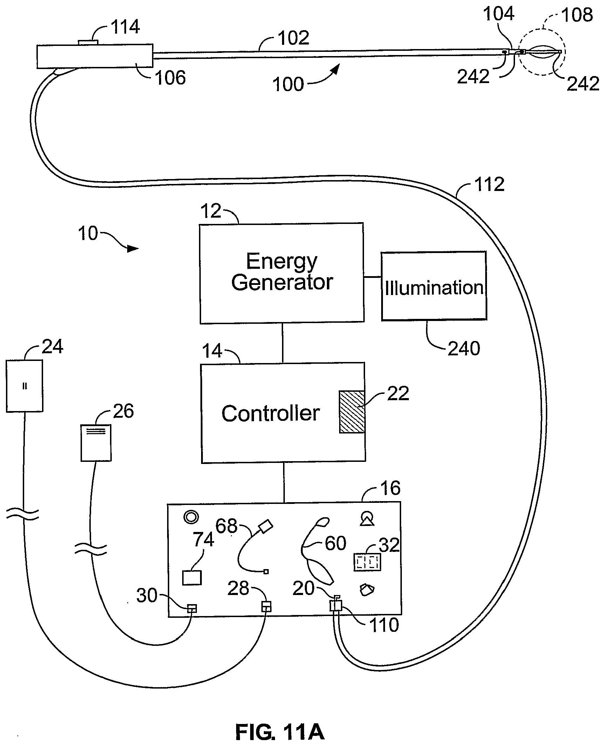

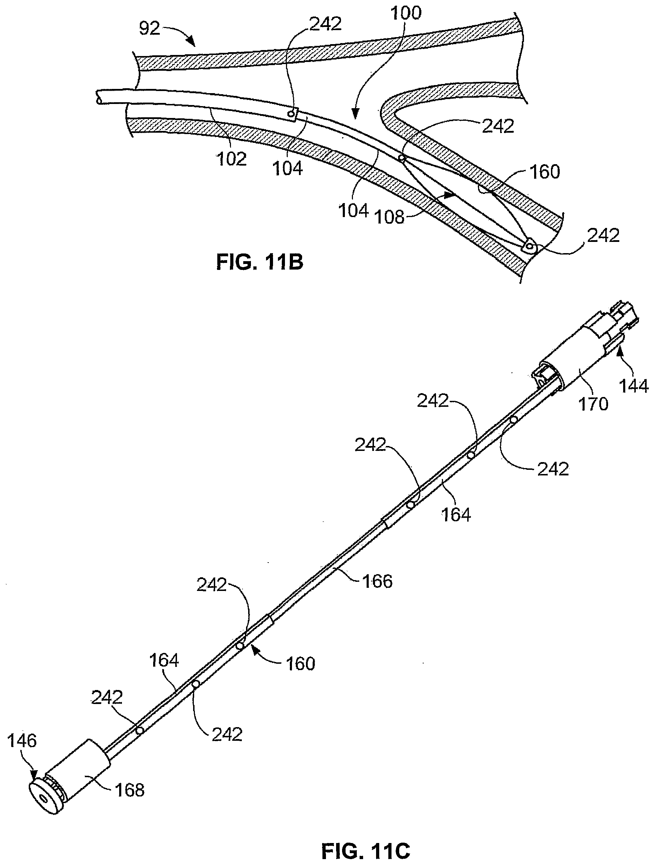

Figs 11A-11C illustrate yet another aspect of the invention in which a treatment device is equipped with an illumination source **242.** As noted above, the illumination source **242** may be configured to provide additional light when the device is used without a scope or to supplement the illumination of the scope. Variations of the invention may include devices having one or more illumination sources **242** that are coupled to an illumination supply **240** that generates the light energy externally to the device (e.g., via a fiber or other type of light conductor). Alternatively, or in combination, the illumination source **242** may generate the light at the distal portion of the device (e.g., via a light emitting diode, etc). It should be noted that although Fig. 11, depicts the illumination supply **240** as being separate from the controller and energy generator, the illumination supply **240** may be integrated into the controller or energy generator.

Fig. 11A illustrates a variation of a system according to the present invention as shown in Fig. 2A above, with the addition of an illumination supply **240** and a device with one or more illumination sources **242.** As noted above, a separate illumination supply **240** is optional as the illumination supply **240** may be incorporated with other components of the system (e.g., device controller, generator, or other).

Fig. 11B shows a variation of a device as shown in Fig. 8A. However, in this variation, the device includes an illumination source **242.** As shown, the illumination source **242** may be located on a tip **122** of the device, on the shaft **104,** on the sheath **102,** on the energy transfer element (as shown in Fig. 11C) or in any combination thereof. Although not illustrated, the illumination source may be placed on or adjacent to a center wire **124** of the basket member **108.** For example, an LED may be placed on the wire, or an optical fiber may be placed adjacent to or wrapped around the wire **124.** Alternatively, a filament or other similar type component may be affixed to the wire in a similar manner, where the filament generates light of a particular wavelength.

Fig. 11C illustrates just one example of a basket leg variation as shown in Fig. 4E above, with the addition of an illumination source **242** that is placed on the leg. In each of the above cases, the illumination source **242** may be an end of a fiber type element that extends through the device and terminates at the illumination source **242** (alternatively or in combination, this variation may include a lens or cover at the termination of the fiber). The illumination source may also comprise a component that emits energy or light (such as a light emitting diode).

It is further contemplated that the illumination source **242** may be placed on a single side of the device or may be placed such that all walls of the airway are illuminated.

It is noted that variations of the device may include a single illumination source **242** or multiple illumination sources **242.** The illumination source(s) **242** may be configured to provide a single or multiple wavelength of light depending upon the particular application. For example, the device may be configured to provide illumination that is visible light, or white light. The illumination can be a single visible color such as red, green, blue, yellow, or a combination. The illumination may be a non-visible wavelength that is made visible by some type of filter or other such means on the scope or viewing monitor for the scope.

Variations of the invention include aiming or positioning the illumination source rearward to aid in light collection by the scope, use of a flex circuit to carry LED and have traces, use of LED lens cap as an atraumatic distal tip.

In addition, certain wavelengths may afford separation from red and orange (e.g., 590 nm, 570 nm, 470 nm or yellow, green, and blue. These colors may offer better distinction when used in airways. In variations of the invention using light emitting diodes (LEDS), the may be commercially available in surface mount configurations having a size that is suited for a device that must fit in a 2 mm working channel. See for example, www.kingbright-led.com, surface mount LED package, APHHS1005.

At the very least, LED may make it easier for the practitioner to identify treated areas within the airway, such as tissue that is blanched or otherwise marked by the application of energy. In these cases, the reflectance of this tissue may be different than surrounding areas.

The invention may also be used with polarizing filters or polarizing fibers to differentiate treated from untreated tissue. Use of circularly polarized filters may be preferred in such a case to eliminate the need for rotation of the filters. In yet another approach the illumination supply/source may use coherent sources of light such as solid state or optical lasers. In the case of a solid state laser, the laser source may actually be placed on the distal end of the device rather than being transmitted via a fiber.

Furthermore, use digital (electronic) filtering of the image from CCD chip mounted at the end of the bronchoscope may permit filtering for desirable wavelengths and/or the image could be amplified to enable discernment. In addition, so long as long the system delivers light containing a broad spectrum of wavelengths, electronic or manual filtering may allow for filtering out any undesirable components. In additional variations, a filter or filters may be placed on the end of the device.

As for other details of the present invention, materials and manufacturing techniques may be employed as within the level of those with skill in the relevant art. The same may hold true with respect to method-based aspects of the invention in terms of additional acts a commonly or logically employed. In addition, though the invention has been described in reference to several examples, optionally incorporating various features, the invention is not to be limited to that which is described or indicated as contemplated with respect to each variation of the invention.

Various changes may be made to the invention described and equivalents (whether recited herein or not included for the sake of some brevity) may be substituted without departing from the scope of the invention.

## Claims

1. A catheter (100) for delivering energy to a body lumen, the catheter comprising:
a flexible elongate shaft having a proximal portion and a distal portion adapted for insertion into a body lumen;
an energy transfer basket (108) coupled to the shaft, the energy transfer basket comprising a plurality of legs adapted to apply energy to the body lumen, each leg having a distal end, a proximal end, and a flexure length that is less than a full length of the leg;
a near alignment component (144) having a plurality of near seats extending along an axis of the alignment component, the near alignment component secured to the elongate shaft; and
a far alignment component (146) having a plurality of far seats extending along an axis of the alignment component, wherein the plurality of near seats are in alignment with the plurality of far seats;
wherein the distal end of each leg is nested within a far seat and the proximal end of each leg is nested within a near seat such that an angle between adjacent legs is determined by an angle between adjacent seats and the flexure length is determined by the distance between near and far alignment components;
wherein the far alignment component includes a distal alignment stop and the near alignment component includes a proximal stop, wherein the distal end of one of the legs contacts the distal stop and the proximal end of the same leg contacts the proximal stop to control the flexure length of each leg, and
wherein the distal alignment stop protrudes radially from a surface of the far alignment component.

2. The catheter of claim 1, wherein the near alignment component comprises a protrusion, and wherein one leg comprises a notched portion which interferes with the protrusion to prevent the leg from moving in a an axial direction.

3. The catheter of claim 1, further comprising a temperature detecting element located on a leg.

4. The catheter of claim 1, wherein the elongate shaft further comprises a fluid delivery or suction lumen.

5. The catheter of claim 1, wherein the leg comprises a bend or shape that predisposes the leg to bow outward.

6. The catheter of claim 1, further comprising a connector for coupling the energy transfer basket to a source of energy for the delivery of monopolar or bipolar radio frequency energy.

7. The catheter of claim 1, adapted for delivering radio frequency energy to an airway wall of a lung,
wherein the flexible elongate shaft is adapted for insertion into an airway of a lung.

## Patentansprüche

1. Katheter (100) zur Abgabe von Energie an ein Körperlumen, wobei der Katheter aufweist:
einen flexiblen länglichen Schaft, der einen proximalen Abschnitt und einen distalen Abschnitt aufweist, der zur Einführung in ein Körperlumen eingerichtet ist;
einen Energieübertragungskorb (108), der mit dem Schaft gekoppelt ist, wobei der Energieübertragungskorb mehrere Schenkel aufweist, die eingerichtet sind, Energie auf das Körperlumen anzuwenden, wobei jeder Schenkel ein distales Ende, ein proximales Ende und eine Biegungslänge aufweist, die kleiner als eine Gesamtlänge des Schenkels ist;
eine nahe Ausrichtungskomponente (144), die mehrere nahe Aufnahmen aufweist, die sich längs einer Achse der Ausrichtungskomponente erstrecken, wobei die nahe Ausrichtungskomponente am länglichen Schaft befestigt ist; und
eine ferne Ausrichtungskomponente (146), die mehrere ferne Aufnahmen aufweist, die sich längs einer Achse der Ausrichtungskomponente erstrecken, wobei die mehreren nahen Aufnahmen mit den mehreren fernen Aufnahmen ausgerichtet sind;
wobei das distale Ende jedes Schenkels in eine ferne Aufnahme gesteckt ist und das proximale Ende jedes Schenkels in eine nahe Aufnahme gesteckt ist, so dass an Winkel zwischen benachbarten Schenkeln durch einen Winkel zwischen benachbarten Aufnahmen festgelegt ist und die Biegungslänge durch den Abstand zwischen der nahen und der fernen Ausrichtungskomponente festgelegt ist;
wobei die ferne Ausrichtungskomponente einen distalen Ausrichtungsanschlag aufweist und die nahe Ausrichtungskomponente einen proximalen Anschlag aufweist, wobei das distale Ende von einem der Schenkel den distalen Anschlag berührt und das proximale Ende desselben Schenkels den proximalen Anschlag berührt, um die Biegungslänge jedes Schenkels zu kontrollieren, und
wobei der distale Ausrichtungsanschlag radial von einer Oberfläche der fernen Ausrichtungskomponente vorsteht.

2. Katheter nach Anspruch 1, wobei die nahe Ausrichtungskomponente einen Vorsprung aufweist, und wobei ein Schenkel einen gekerbten Abschnitt aufweist, der mit dem Vorsprung in Eingriff steht, um den Schenkel an einer Bewegung in eine axiale Richtung zu hindern.

3. Katheter nach Anspruch 1, der ferner ein Temperaturermittlungselement aufweist, das an einem Schenkel angeordnet ist.

4. Katheter nach Anspruch 1, wobei der längliche Schaft ferner ein Fluidzufuhr- /oder Sauglumen aufweist.

5. Katheter nach Anspruch 1, wobei der Schenkel eine Biegung oder Form aufweist, die den Schenkel prädisponiert, sich nach außen durchzubiegen.

6. Katheter nach Anspruch 1, der ferner einen Steckverbinder zur Kopplung des Energieübertragungskorbs mit einer Energiequelle zur Abgabe von monopolarer oder bipolarer Hochfrequenzenergie aufweist.

7. Katheter nach Anspruch 1, der zur Abgabe von Hochfrequenzenergie an eine Atemwegwand einer Lunge eingerichtet ist,
wobei der flexible längliche Schaft zur Einführung in einen Atemweg einer Lunge eingerichtet ist.

## Revendications

1. Cathéter (100) pour l'application d'énergie dans une lumière corporelle, ledit cathéter comprenant :
une tige allongée flexible ayant une partie proximale et une parie distale, prévue pour être insérée dans une lumière corporelle ;
un panier de transfert d'énergie (108) raccordé à la tige, ledit panier de transfert d'énergie comprenant une pluralité de bras prévus pour une application d'énergie dans la lumière corporelle, chaque bras ayant une extrémité distale, une extrémité proximale et une longueur de flexion inférieure à la longueur totale du bras ;
un élément d'alignement proche (144) ayant une pluralité de segments proches s'étendant le long d'un axe de l'élément d'alignement, ledit élément d'alignement proche étant fixé sur la tige allongée ; et
un élément d'alignement distant (146) ayant une pluralité de segments distants s'étendant le long d'un axe de l'élément d'alignement, la pluralité de segments proches étant alignée avec la pluralité de segments distants ;
où l'extrémité distale de chaque bras est logée dans un segment distant et l'extrémité proximale de chaque bras est logée dans un segment proche, de sorte qu'un angle entre bras adjacents est déterminé par un angle entre segments adjacents, et que la longueur de flexion est déterminée par la distance entre éléments d'alignement proches et éléments d'alignement distants ;
où l'élément d'alignement distant comprend une butée d'alignement distale et l'élément d'alignement proche une butée proximale, l'extrémité distale d'un des bras contactant la butée distale, et l'extrémité proximale du même bras contactant la butée proximale pour commander la longueur de flexion de chaque bras, et
où la butée d'alignement distale fait saillie radialement d'une surface de l'élément d'alignement distant.

2. Cathéter selon la revendication 1, où l'élément d'alignement proche présente une saillie, et où un bras comprend une partie crantée coopérant avec la saillie pour empêcher le bras de se déplacer en direction axiale.

3. Cathéter selon la revendication 1, comprenant en outre un élément détecteur de température disposé sur un bras.

4. Cathéter selon la revendication 1, où la tige allongée comprend en outre une lumière d'administration de fluide ou d'aspiration.

5. Cathéter selon la revendication 1, où le bras présente une courbure ou une forme prédisposant le bras à fléchir vers l'extérieur.

6. Cathéter selon la revendication 1, comprenant en outre un connecteur pour le branchement du panier de transfert d'énergie sur une source d'énergie pour l'application d'une énergie à fréquence radio monopolaire ou bipolaire.

7. Cathéter selon la revendication 1, prévu pour l'application d'une énergie à fréquence radio à une paroi de voie aérienne d'un poumon,
où la tige allongée flexible est prévue pour être insérée dans une voie aérienne d'un poumon.
